# EUROPÄISCHE PATENTSCHRIFT

(11) **EP 2 040 774 B1**
(45) Veröffentlichungstag und Bekanntmachung des Hinweises auf die Patenterteilung: **27.08.2014**
(21) Anmeldenummer: 07763937.5
(22) Anmeldetag: 10.07.2007
(51) Int. Cl.: A61M 1/06

(54) **BRUSTPUMPENSET**
BREASTPUMP SET
ENSEMBLE DE POMPE MAMMAIRE

(30) Priorität: 18.07.2006 CH 11562006
(43) Veröffentlichungstag der Anmeldung: 01.04.2009
(73) Patentinhaber: Medela Holding AG, 6340 Baar (CH)
(72) Erfinder: BOSSHARD, Patrik, CH-6343 Buonas (CH); WEBER, Beda, CH-5643 Sins (CH); GIEZENDANNER, Charles, CH-6443 Morschach (CH)
(74) Vertreter: Clerc, Natalia
(86) Internationale Anmeldenummer: PCT/CH2007/000332
(87) Internationale Veröffentlichungsnummer: WO 2008/009145

(56) Entgegenhaltungen:
- EP-A- 1 502 610
- WO-A-00/41744
- WO-A-03/066133
- WO-A-2006/079229
- US-A1- 2002 182 584
- US-B1- 6 547 756

## Beschreibung

### Technisches Gebiet

Die Erfindung betrifft ein Brustpumpenset, eine Brusthaube und ein Verfahren zur Betreibung eines Brustpumpensets.

### Stand der Technik

Brustpumpen zum Abpumpen von menschlicher Muttermilch sind hinlänglich bekannt. Grundsätzlich gibt es zwei verschiedene Typen: der erste wird manuell bedient, d.h. der für das Abpumpen notwendige Unterdruck wird durch manuelle Betätigung der Saugpumpe erzeugt. Beim zweiten Typ ist die Saugpumpe elektrisch betrieben, wobei die Saugpumpe ans Stromversorgungsnetz angeschlossen sein kann und/oder über eine Batterie bzw. einen anderen Energiespeicher betrieben werden kann.

Damit die Funktion der Brustpumpe optimal an die Bedürfnisse der Mutter angepasst werden kann, bieten einige der Pumpen der Mutter die Möglichkeit, den Unterdruck zu regulieren. So offenbaren US 2004/0024351 und US 4 813 932 manuell betätigte Brustpumpen, welche einstellbare Ventile aufweisen. Die Saugleistung der Pumpe selber wird bei manuell betriebenen Pumpen zudem dadurch geregelt, dass die Mutter entsprechend ihren Bedürfnissen die Pumpe stärker oder schwächer betätigt.

Auch bei elektrisch betriebenen Pumpen ist es üblich, an der Brusthaube oder an der Verbindungsleitung zur Saugpumpe Entlüftungsöffnungen anzubringen. Dies ist beispielsweise in US 6 706 012 und US 6 042 560 der Fall.

US 6 110 140 schlägt eine manuell oder elektrisch betriebene Brustpumpe vor, die im Bereich der Brusthaube einen Vakuumregulator aufweist, welcher den durch die Saugpumpe erzeugten Unterdruck reguliert. Dieser Regulator lässt sich während des Gebrauchs der Brustpumpe betätigen, so dass der in der Brusthaube herrschende Unterdruck angepasst werden kann.

Zusätzlich zu den oben beschriebenen Lösungen, welche alle den von der Saugpumpe erzeugten Unterdruck im Bereich der Brusthaube ändern können, verfügen einige elektrisch betriebene Brustpumpen zudem über die Möglichkeit, den Unterdruck bzw. den Saugrhythmus am Pumpengerät selber zu ändern. Dies erfolgt über entsprechende Betätigungsschalter oder -knöpfe, welche an der Saugpumpe angeordnet sind. Um diese Knöpfe betätigen zu können, muss die Mutter jedoch eine Hand frei haben. Dies ist jedoch insbesondere dann nicht, oder nur erschwert, möglich, wenn die Mutter beide Brüste gleichzeitig abpumpen möchte.

Aus US 6 547 756 ist eine programmierbare Brustpumpe bekannt, welche entsprechend verschiedener Pumpprogramme die für die Mutter und den Säugling optimale Saugleistung und Pumpfrequenz in Abhängigkeit der Zeit einstellen und zur Verfügung stellen kann. Die Programme werden vorzugsweise von einem Computer in eine Speichereinheit der Pumpe geladen und/oder auf einem von der Elektronik der Pumpe lesbaren Speicherchip geladen.

EP 1 502 610 schlägt vor, eine handbetriebene Brustpumpe mit einer elektronischen Brustpumpe zu kombinieren. Über den Handbetrieb wird die aktuell gewünschte Pumpfrequenz in einem Lernmodus an die Elektronik der elektronischen Brustpumpe weitergegeben. Bei Umschalten auf Automatikbetrieb arbeitet die Pumpe nach den im Lernmodus vorgegebenen Werten.

In der WO 2005/070476 wird ein Brusthaubeneinsatz zum Einlegen in eine Brusthaube beschrieben, welcher integrierte elektrische Heizelemente aufweist. Dadurch lässt sich die Brusthaube auf eine für die Mutter angenehme Temperatur erwärmen.

In der WO 2003/066133 wird eine Brusthaube vorgeschlagen, welche an vorbestimmten Stellen mit Messelektroden ausgerüstet ist. Diese Elektroden sind mit einem Auswertungsgerät verbunden, welches in der Brustpumpe integriert sein kann.

In der noch unveröffentlichten PCT Anmeldung PCT/CH 2005/000730 (WO 2 006 079 229) vom 07. Dezember 2005 wird ferner vorgeschlagen, eine Brusthaube mit Bedienungsmitteln zur Bedienung der Saugpumpe zu versehen. Dadurch kann die Mutter während des Abpumpvorgangs die Funktion der Saugpumpe beeinflussen, ohne dass diese für sie erreichbar sein muss. Die Mutter kann zur Bedienung der Bedienungsmittel dieselbe Hand verwenden, mit welcher sie die Brusthaube an ihre Brust hält. Dies ist insbesondere beim gleichzeitigen Abpumpen von beiden Brüsten vorteilhaft.

In der WO 00/41744 wird eine Vorrichtung zum Abpumpen von menschlicher Muttermilch beschrieben. wobei die Vorrichtung eine Brusthaube sowie eine elektrisch betriebene Saugpumpe umfasst An der Saugpumpe ist eine Benutzerbedienungsfläche angeordnet.

In der US 2002/0182584 ist eine Vorrichtung zur Aspiration von Brustflüssigkeit gezeigt, bei welcher in einem ersten Ausführungsbeispiel für den Heimgebrauch ein Brusthaubentrichter direkt an einem Saugpumpengehäuse angebracht ist. In einem zweiten Ausführungsbeispiel für den Gebrauch zum Beispiel in einem Spital ist der Brusthaubentrichter über eine Saugleitung mit dem Saugpumpengehäuse verbunden. In beiden Fällen weist das Saugpumpengehäuse auf seiner Aussenseite jeweils Kontrollelemente zum Bedienen der Saugpumpe auf.

### Darstellung der Erfindung

Es ist deshalb eine Aufgabe der Erfindung, der Mutter das Abpumpen der Muttermilch so einfach und doch so komfortabel wie möglich zu gestalten.

Diese Aufgabe löst ein Brustpumpenset mit den Merkmalen des Patentanspruchs 1, eine Brusthaube mit den Merkmalen des Patentanspruchs 15 sowie ein Verfahren mit den Merkmalen des Patentanspruchs 13.

Erfmdungsgemäss ist brusthaubenseitig ein Sender und/optional ein Empfänger vorhanden, so dass die Mutter während des Abpumpens brusthaubenseitig Aktionen auslösen und/oder steuern und/oder überwachen und/oder Informationen erhalten kann. Hierzu benötigt sie keine freie Hand, sondern kann diejenige Hand verwenden, welche die Brusthaube an die Brust hält. Zudem kann die Brusthaube automatisch manuell erzeugte Pumpvorgänge oder -moden an einen Speicher oder eine Steuerung einer Saugpumpeneinheit oder an ein anderes Speichermedium übermitteln.

Vorzugsweise erfolgt die Kommunikation von der brusthaubenseitigen Sende- und optionalen Empfangseinheit drahtlos. Die uni- oder bidirektionale Kommunikation erfolgt vorzugsweise mit einer Saugpumpeneinheit bzw. mit einer Steuerung der Saugpumpe. Sie kann jedoch zusätzlich mit einem Computer oder einem sonstigen Datenverarbeitungsgerät oder auch mit dem Milchsammelbehälter erfolgen. Im letzten Fall kann zum Beispiel im Milchsammelbehälter ein Füllstandsensor angeordnet sein, welcher den Füllstand an den brusthaubenseitigen Empfänger und/oder an den saugpumpenseitigen Empfänger meldet. Des weiteren kann der brusthaubenseitige Empfänger eine Funkuhr oder eine andere Zeitmessung enthalten, mittels welcher die Pumpzeit angezeigt wird. Die Uhrzeit bzw. die Pumpdauer kann auch als Signal von der Saugpumpeneinheit an den brusthaubenseitigen Empfänger übermittelt werden.

Die drahtlose Kommunikation hat den Vorteil, dass keine Verbindungsstecker, Anschlüsse und Kabel notwendig sind, welche schnell verschmutzen können und aufwendig zu reinigen sind. Des weiteren reduzieren sich dadurch die Herstellungskosten, was insbesondere für die Brusthauben, welche aus hygienischen Gründen nur während einer kurzen Zeit und nur von einer einzigen Person verwendet werden sollten, relevant ist.

Das erfindungemässe Brustpumpenset zum Abpumpen von menschlicher Muttermilch weist mindestens eine Brusthaube zur Anlage an eine Mutterbrust mit einem Kopplungsteil zur Verbindung mit einem Milchauffangbehälter und eine Saugpumpeneinheit mit einer elektrisch betriebenen Saugpumpe auf, wobei brusthaubenseitig eine Signalsendeeinheit angeordnet ist zur Übermittlung von Signalen an die Saugpumpeneinheit. Diese Signalsendeeinheit umfasst erfindungsgemäss eine Programmierungsvorrichtung zur manuellen oder automatischen Programmierung der Saugpumpeneinheit, die dazu ausgebildet ist, einen gespeicherten Pumpvorgang an die Saugpumpeneinheit zu übermitteln.

In einer weiteren bevorzugten Ausführungsform weist das erfindungsgemässe Brustpumpenset zum Abpumpen von menschlicher Muttermilch zwei Signalsendeeinheiten auf wobei eine erste Signalsendeeinheit brusthaubenseitig und einen zweite Signalsendeeinheit saugpumpenseitig angeordnet ist.

In einer weiteren bevorzugten Ausführungsform ist im Bereich der Brusthaube eine Signalempfängereinheit angeordnet.

Vorzugsweise ist die brusthaubenseitige Signalsende- und optionale Empfangseinheit der oben beschriebenen drei Ausführungsformen an der Brusthaube, an einer brusthaubenseitigen Steckverbindung eines Vakuum- oder Saugschlauches oder am Vakuumschlauch selber angebracht.

Weitere vorteilhafte Ausführungsformen gehen aus den abhängigen Patentansprüchen hervor.

### Kurze Beschreibung der Zeichnungen

Im folgenden wird der Erfindungsgegenstand anhand von bevorzugten Ausführungsbeispielen, welche in den beiliegenden Zeichnungen dargestellt sind, erläutert. Es zeigen:
- Figur 1: eine schematische Darstellung eines Brustpumpensets gemäss einer ersten Ausführungsform der Erfindung;
- Figur 2: eine schematische Darstellung eines Brustpumpensets gemäss einer zweiten Ausführungsform der Erfindung;
- Figur 3: eine schematische Darstellung eines Brustpumpensets gemäss einer dritten Ausführungsform der Erfindung;
- Figur 4: eine perspektivische Darstellung einer erfindungsgemässen Brusthaube gemäss einer vierten Ausführungsform der Erfindung;
- Figur 5: eine erste Variante des Brustpumpensets gemäss Figur 1 und
- Figur 6: eine zweite Variante des Brustpumpensets gemäss Figur 1.

### Wege zur Ausführung der Erfindung

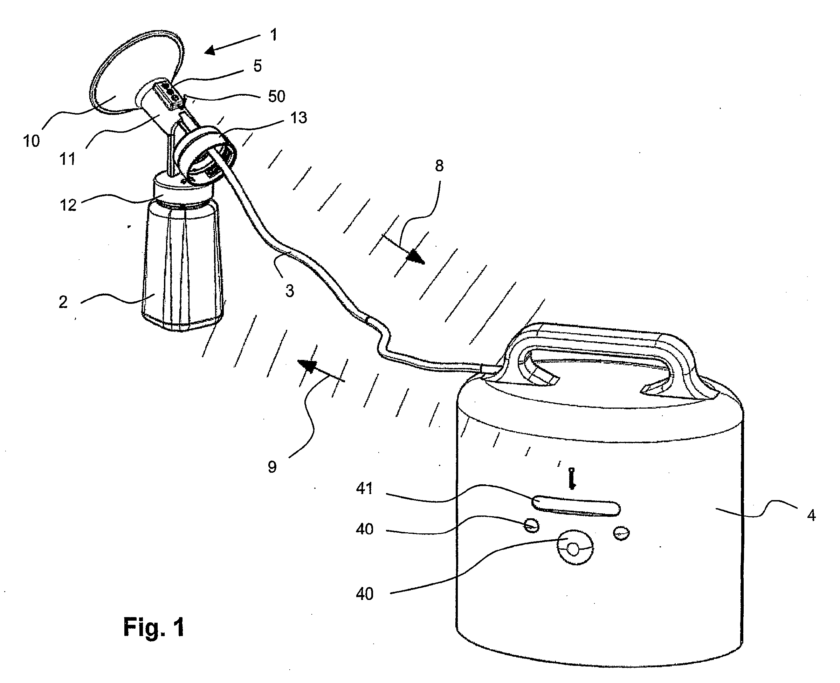

In Figur 1 ist eine erste Ausführungsform des erfindungsgemässen Brustpumpensets dargestellt. Es weist im wesentlichen mindestens eine Brusthaube 1, einen an die Brusthaube 1 befestigbaren Milchauffang- oder -sammelbehälter 2 und eine Saugpumpeneinheit 4 mit einer elektrisch betriebenen Saugpumpe auf, welche über eine Saugleitung 3 mit der Brusthaube 1 verbindbar ist. Die Verbindung erfolgt vorzugsweise über Steckverbindungen.

Werden zwei Brusthauben 1 verwendet, um beide Brüste gleichzeitig abzupumpen, so sind zwei Saugleitungen vorhanden, welche an dasselbe Pumpgerät angeschlossen werden.

Die Brusthaube 1 weist einen Brusthaubentrichter 10 auf, welcher bei Gebrauch an die Brust der Mutter angelegt wird. Der Trichter 10 geht in einen Brusthaubenhals 11 über, an welchem ein erstes und ein zweites Brusthaubenkopplungsteil 12, 13 angrenzen. Das erste Brusthaubenkopplungsteil 12 weist ein Innengewinde auf, welches sich auf ein Aussengewinde des Milchsammelbehälters 2, hier eine Babyflasche, aufschrauben lässt. Das zweite Brusthaubenkopplungsteil 13 weist ein in der Figur nicht sichtbares Anschlussteil auf, in welches die Saugleitung 3, hier ein Schlauch, einsteckbar ist. Das andere Ende des Schlauchs ist in der Saugpumpe 4 einsteckbar. Über diesen Schlauch 3 wird das in der Saugpumpe erzeugte Vakuum bzw. der dort erzeugte Unterdruck an die Brusthaube angelegt, so dass die Milch aus der Mutterbrust abgepumpt und im Milchsammelbehälter 2 gesammelt werden kann.

In Figur 1 ist die übliche Gebrauchslage dargestellt. Das erste Kopplungsteil 12 ist nach unten gerichtet, das zweite Kopplungsteil 13 nach hinten oder schräg nach unten, auf jeden Fall von der Mutterbrust weg.

Als Saugpumpeneinheit 4 lassen sich alle bekannten motorisch angetriebenen Geräte verwenden. Vorzugsweise wird jedoch eine Saugpumpe der Anmelderin verwendet, welche unter dem Handelsnamen Symphony bekannt ist. Vorzugsweise verfügt die Saugpumpeneinheit über Betätigungsknöpfe und -tasten 40 und eine Anzeige 41. Im hier dargestellten Ausführungsbeispiel ist sie als eigenständiges portables Gerät ausgebildet. Sie kann jedoch auch an der Brusthaube angebracht sein, so dass die Saugleitung 3 entfällt. Auch derartige Geräte sind auf dem Markt bekannt.

Erfindungsgemäss ist nun brusthaubenseitig eine Sende- und/optionale Empfangseinheit 5 vorhanden, welche in diesem Beispiel über eine Antenne 50 zum Senden und/optional Empfangen von Signalen verfügt. Die Antenne 50 ist hier dem Gehäuse der restlichen Einheit vorstehend dargestellt. Sie kann jedoch auch im Gehäuse angeordnet und insbesondere flächig ausgebildet sein. Anstelle einer Funkantenne lassen sich auch andere Mittel zur drahtlosen Kommunikation verwenden, beispielsweise eine Infrarotschnittstelle, Bluetooth, optische oder akustische Signale, ein passives Resonanzverstimmelement, RFID oder andere bekannte Mittel.

Die Sende- und Empfangseinheit 5 verfügt über Bedienungstasten 51, 52, 53, welche vorzugsweise so angeordnet sind, dass sie für die Mutter einfach zugänglich und betätigbar sind, ohne dass sie ihre die Tasten betätigende Hand von der Brusthaube lösen muss. Anstelle der Tasten lassen sich auch Drehknöpfe, induktive Tastfelder, Schieber, Schalter oder andere Bedienungselemente bekannter Art verwenden.

Des weiteren kann die Sende- und Empfangseinheit mit einer optischen und/oder akustischen Anzeige ausgestattet sein. Über die Anzeige lassen sich beispielsweise Statusinformationen anzeigen, wie die Pumpfrequenz und Pumpleistung, das ausgewählte Pumpprogramm oder ob gerade programmiert wird. Es lassen sich auch die diversen Optionen der Saugpumpe, wie beispielsweise die zur Verfügung stehenden Pumpkurvenverläufe, anzeigen, um eine entsprechende Wahl treffen zu können.

Ferner kann die Anzeige über einen akustischen Signalgeber verfügen, beispielsweise einen Alarm zur Kenntnismachung einer falschen Bedienung einzelner Elemente des Sets und/oder einem Tongeber zur Bekanntmachung eines Versands oder Empfangs von Signalen.

Die Sende- und Empfangseinheit 5 ist vorzugsweise im Bereich des Brusthaubentrichters 10 angeordnet. Im hier dargestellten Beispiel ist sie angrenzend an den Trichter 10 auf dem Brusthaubenhals 11 befestigt bzw. in diesem eingelassen. Vorzugsweise ist sie in der hier dargestellten Gebrauchslage nach oben gerichtet, so dass sie mit derselben Hand erreichbar ist, mit welcher die Mutter die Brusthaube 1 an ihre Brust hält. Die Einheit 5 kann jedoch beispielsweise auch seitlich oder unten am Hals 11 angeordnet sein.

Die Befestigung der Einheit 5 auf oder in der Brusthaube 1 erfolgt mittels bekannten Mitteln. Der Grundkörper der Brusthaube 1 ist selber üblicherweise aus Kunststoff gefertigt und ein- oder mehrstückig ausgebildet. Die Einheit 5 kann beispielsweise in diesen Grundkörper eingegossen sein oder auf ihm aufgeklebt sein. Im zweiten Fall kann der Grundkörper eine entsprechende Ausnehmung zur steckbaren Aufnahme der Einheit 5 aufweisen, damit diese nicht allzu sehr vorsteht und somit geschützt ist. Allfällige Kabel zur Signalübertragung lassen sich auf dieselbe Weise mit der Brusthaube 1 verbinden bzw. in diese einlassen.

Erfindungsgemäss kann die Einheit 5 eine reine Signalsendeeinheit ohne Empfangsfunktion, oder eine kombinierte Signalsende- und -empfangseinheit sein. Vorzugsweise verfügt auch die Saugpumpeneinheit über eine Signalsende- und/oder Signalempfangseinheit. Auch hier sind die drei oben genannten Kombinationen möglich, wobei sie in Kombination mit der Einheit 5 zu einer funktionierenden uni- oder bidirektionalen Kommunikation führen sollen. Das heisst, ist der eine ein reiner Sender, so kann der andere die vom ersten übermittelten Signale empfangen usw. Selbstverständlich verfügt auch die Saugpumpeneinheit 4 über eine Antenne oder eine andere geeignete Schnittstelle zur drahtlosen Kommunikation.

In Figur 1 ist die Kommunikation bidirektional. Die brusthaubenseitigen Signale sind mit der Bezugsziffer 8 und die saugpumpenseitigen Signale sind mit der Bezugsziffer 9 versehen.

Grundsätzlich sind folgende Varianten möglich, wobei dies nicht nur für dieses Ausführungsbeispiel sondern auch für die nachfolgend beschriebenen Beispiele sowie für alle Ausführungsformen gemäss der Erfindung gilt:

Es ist brusthaubenseitig eine Signalsendeeinheit angeordnet zur Übermittlung von Signalen an die Saugpumpeneinheit, wobei die Signalsendeeinheit eine Programmierungsvorrichtung zur manuellen oder automatischen Programmierung der Saugpumpeneinheit umfasst. Die Programmierungsvorrichtung übermittelt dabei einen manuellen Pumpvorgang an die Saugpumpeneinheit. Sie kann zusätzlich oder anstatt auch einen gespeicherten Pumpvorgang an die Saugpumpeneinheit übermitteln und in der Saugpumpeneinheit aktivieren.

Es können zwei reine Signalsendeeinheiten vorhanden sein, wobei eine erste Signalsendeeinheit brusthaubenseitig und eine zweite Signalsendeeinheit saugpumpenseitig angeordnet ist. Entsprechende Signale können zur Anzeige von Informationen im Bereich der Brusthaube gebracht werden oder auch beispielsweise an Datenverarbeitungseinheiten zur Datenauswertung gesendet werden.

Es kann auch im Bereich der Brusthaube eine Signalempfängereinheit angeordnet sein, wobei die Saugpumpeneinheit und/oder ein externes Datenverarbeitungsgerät über einen entsprechenden Sender verfügt.

Die Signaleinheit kann über Bedienungsmittel zur Bedienung der Saugpumpe verfügen, beispielsweise um die Pumpe ein- und abzuschalten, um die Frequenz, die Pumpleistung oder um ein spezielles Pumpprogramm zu wählen. Es lässt sich ein manueller Pumpmodus oder ein mittels des Bedienungsmittels programmierter Pumpmodus übertragen.

Die Brustpumpe kann mit einer Signalsendeeinheit zur Sendung von Signalen an die brusthaubenseitige Signalempfängereinheit ausgerüstet sein. Die Brustpumpe kann mit einer Signalsende- und Signalempfangseinheit zur Sendung und zum Empfang von Signalen an die brusthaubenseitige Signalempfänger- und Sendeeinheit bzw. von der brusthaubenseitigen Signalempfänger- und Sendeeinheit ausgerüstet sein.

Die übermittelten Informationen können beispielsweise eine oder mehrere aus folgender Gruppe sein: Saugfrequenz, Saugleistung, verwendetes Pumpprogramm, zur Auswahl stehende Pumpprogramme, Pumpdauer, Uhrzeit, Füllstand im Milchsammelbehälter, Brusthaubentemperatur, Milchflussmessung. Es lassen sich beispielsweise auch Teile der Brusthaube damit steuern oder regeln, wie beispielsweise Entlüftungsventile.

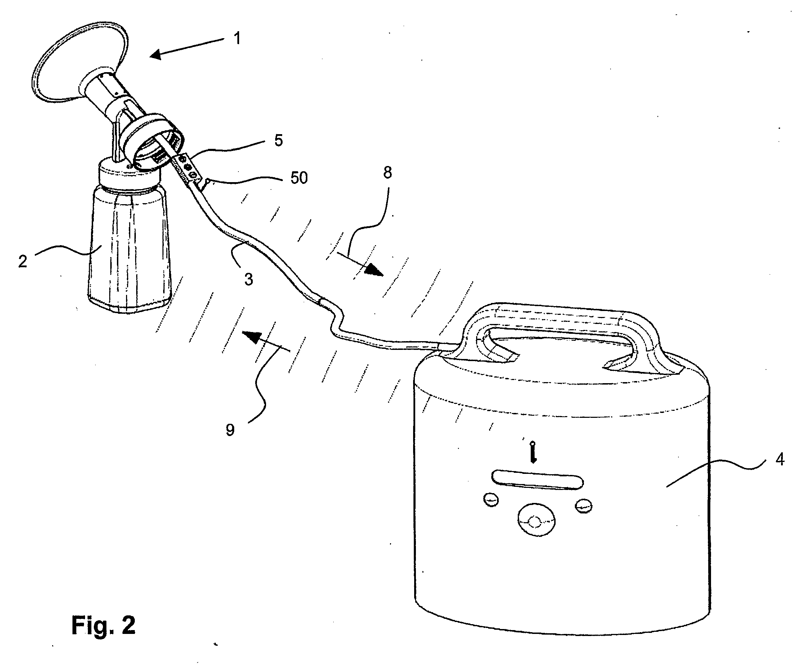

In Figur 2 ist nun ein zweites Ausführungsbeispiel dargestellt. Gleiche Teile sind hier und in den nachfolgenden Beispielen mit gleichen Bezugszeichen versehen und werden deshalb nicht mehr wiederholt. Die brusthaubenseitige Sende- und/optionale Empfangseinheit 5 ist in diesem Beispiel nun nicht mehr an der Brusthaube 1 sondern an der Saugleitung 3 angeordnet. Sie kann in einer weiteren Variante auch an einer Steckverbindung zwischen Brusthaube 1 und Saugleitung 3 angeordnet sein.

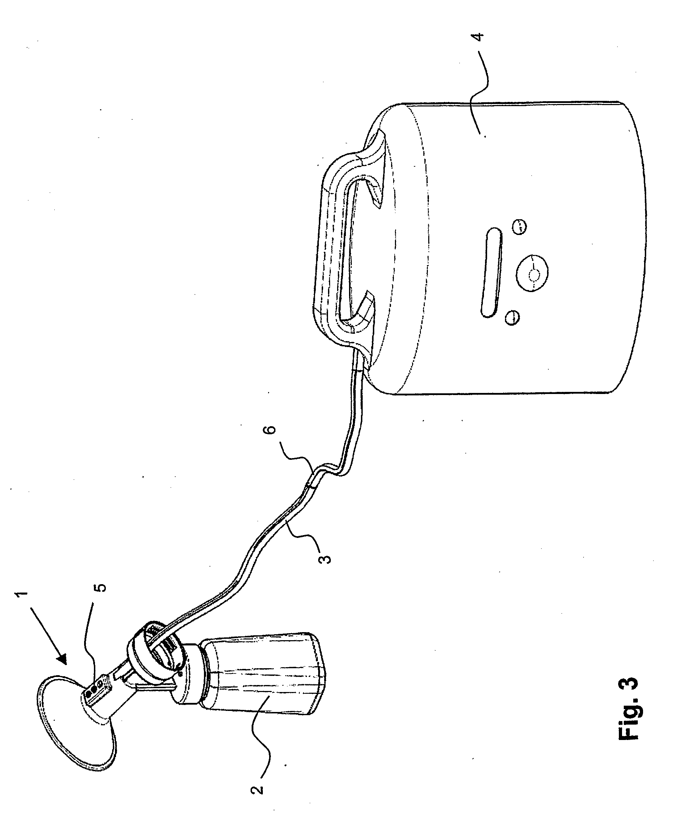

Im Ausführungsbeispiel gemäss Figur 3 ist nun keine drahtlose Kommunikation sondern mindestens eine analoge oder digitale Datenleitung 6 zur uni- bzw. bidirektionalen Übermittlung der Signale vorhanden, welche vorzugsweise an der Saugleitung 3 befestigt ist.

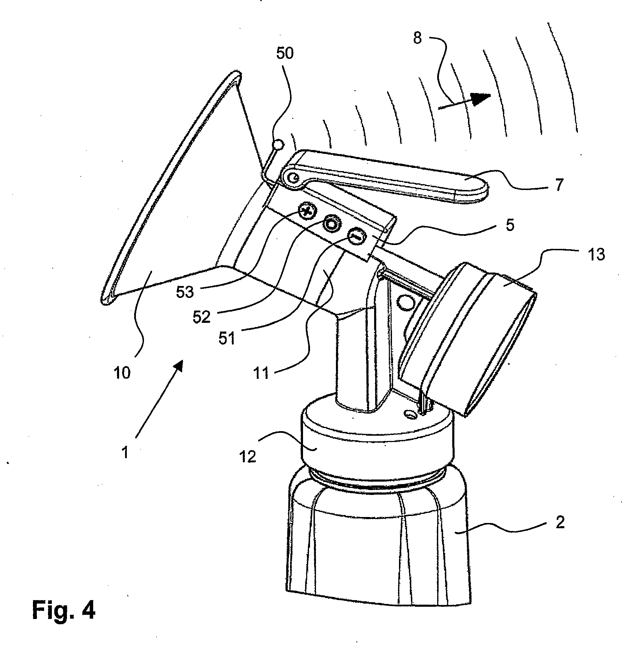

In Figur 4 ist eine Variante einer Brusthaube 1 dargestellt, welche sich in allen Ausführungsformen einsetzen lässt. Sie verfügt über einen Betätigungsschalter oder -hebel 7, über den manuell durch die Mutter ein Pumpmodus oder -rhythmus (zeitlicher Verlauf des Vakuums) vorgegeben werden kann, dessen Signale an die Saugpumpeneinheit 4 übermittelt werden.

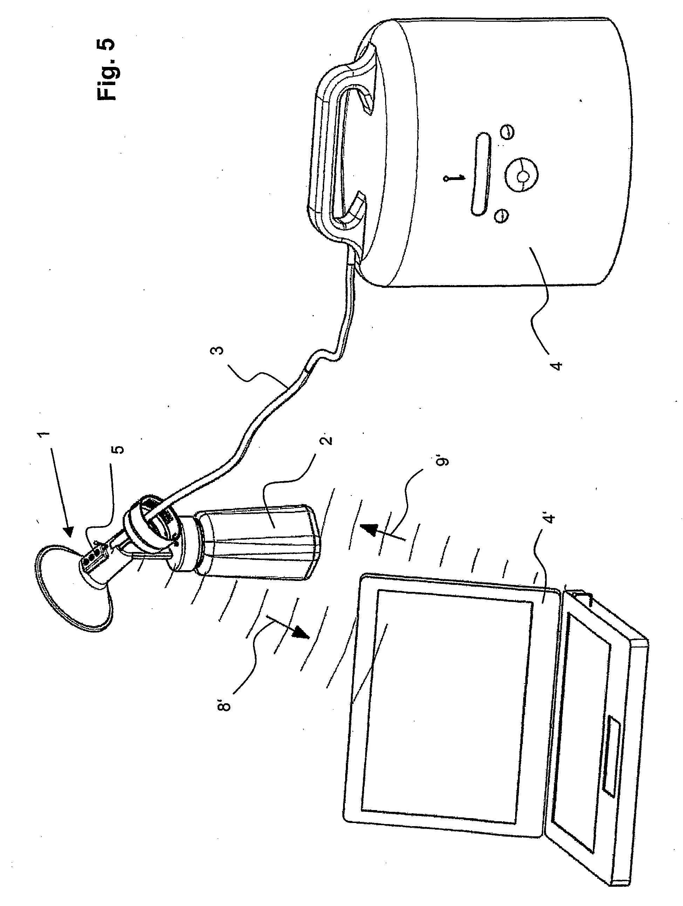

Figur 5 zeigt eine bidirektionale Kommunikation 8', 9' mit einem externen Gerät 4', hier einem Computer. Die Kommunikation könnte aber auch hier unidirektional sein, wobei die dargestellte Richtung 8' hierfür in Frage kommt. Anstelle des Computers lassen sich auch andere bekannte Geräte, wie ein Handheld, ein Mobilphon, eine Funkuhr, eine frei programmierbare Fernbedienung und ähnliches einsetzen.

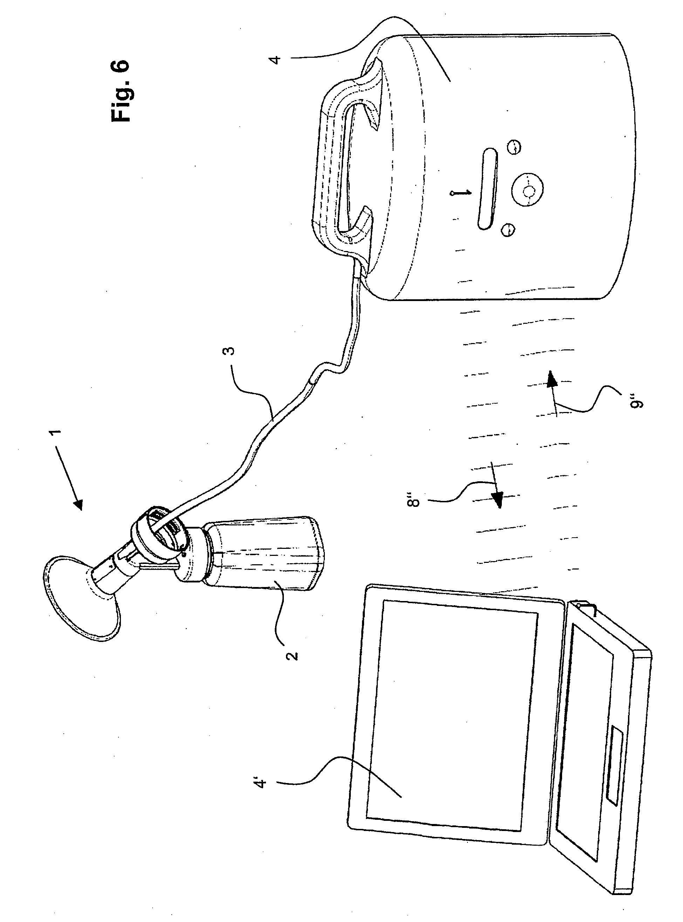

In Figur 6 findet die Kommunikation 8", 9" zwischen externem Gerät 4' und Saugpumpeneinheit 4 statt. Selbstverständlich können die Kommunikationen auch gleichzeitig oder nacheinander zwischen externem Gerät und Brusthaube, externem Gerät und Saugpumpeneinheit und Brusthaube und Saugpumpeneinheit stattfinden. In dieser Figur ist die Sender- und Empfängereinheit 5 nicht dargestellt. Sie kann selbstverständlich vorhanden sein oder aber bei der Kommunikation zwischen den zwei oben genannten Geräten auch fehlen.

Das erfindungsgemässe Brustpumpenset erleichtert der Mutter während des Abpumpens die Bedienung der Pumpe und der Brusthaube.

### Bezugszeichenliste

- 1: Brusthaube
- 10: Brusthaubentrichter
- 11: Brusthaubenhals
- 12: erstes Brusthaubenkopplungsteil
- 13: zweites Brusthaubenkopplungsteil

- 2: Milchauffangbehälter

- 3: Saugleitung

- 4: Saugpumpeneinheit
- 4': Computer
- 40: Betätigungstasten bzw. -knöpfe
- 41: Anzeige

- 5: Sender- und/oder Empfängereinheit
- 50: Antenne
- 51: Einschaltknopf
- 52: erster Betätigungsknopf
- 53: zweiter Betätigungsknopf

- 6: Signalleitung

- 7: Betätigungshebel

- 8: brusthaubenseitige Signale
- 8': brusthaubenseitige Signale
- 8": saugpumenseitige Signale

- 9: saugpumpenseitige Signale

- 9': computerseitige Signale
- 9": computerseitige Signale

## Patentansprüche

1. Brustpumpenset zum Abpumpen von menschlicher Muttermilch, wobei das Brustpumpenset mindestens eine Brusthaube (1) zur Anlage an eine Mutterbrust mit einem Kopplungsteil (12) zur Verbindung mit einem Milchauffangbehälter (2) und eine Saugpumpeneinheit (4) mit einer elektrisch betriebenen Saugpumpe aufweist, wobei brusthaubenseitig eine erste Signalsendeeinheit (5) angeordnet ist zur Übermittlung von Signalen an die Saugpumpeneinheit (4), und wobei diese erste Signalsendeeinheit (5) eine Programmierungsvorrichtung zur manuellen oder automatischen Programmierung der Saugpumpeneinheit (4) umfasst, **dadurch gekennzeichnet, dass** die Programmierungsvorrichtung dazu ausgebildet ist, einen gespeicherten Pumpvorgang an die Saugpumpeneinheit (4) zu übermitteln.

2. Brustpumpenset nach Anspruch 1, wobei die Programmierungsvorrichtung einen manuellen Pumpvorgang erfasst und diesen an die Saugpumpeneinheit (4) übermittelt und/oder wobei die Programmierungsvorrichtung einen gespeicherten Pumpvorgang in der Saugpumpeneinheit (4) aktiviert.

3. Brustpumpenset nach einem der Ansprüche 1 oder 2, wobei die erste Signalsendeeinheit (5) zur drahtlosen Übermittlung der Signale ausgebildet ist.

4. Brustpumpenset nach einem der Ansprüche 1 bis 3, wobei das Brustpumpenset zwei Signalsendeeinheiten aufweist, und wobei die erste Signalsendeeinheit (5) brusthaubenseitig und eine zweite Signalsendeeinheit saugpumpenseitig angeordnet ist.

5. Brustpumpenset nach Anspruch 4, wobei die zwei Signalsendeeinheiten zur drahtlosen Übermittlung von Signalen ausgebildet sind und/oder wobei die brusthaubenseitige Signalsendeeinheit (5) vorzugsweise an der Brusthaube (1), an einer brusthaubenseitigen Steckverbindung zu einem Saugschlauch (3) zwischen Brusthaube (1) und Saugpumpe (4) oder am Saugschlauch (3) angeordnet ist.

6. Brustpumpenset nach einem der Ansprüche 1 bis 5, wobei im Bereich der Brusthaube (1) eine Signalempfängereinheit (5) angeordnet ist.

7. Brustpumpenset nach Anspruch 6, wobei die Signalempfängereinheit (5) an oder in der Brusthaube (1) angeordnet ist oder wobei für jede Brusthaube (1) eine Saugleitung (3) vorhanden ist zur Verbindung der Saugpumpe (4) mit der mindestens einen Brustpumpe und wobei die Signalempfängereinheit (5) an der Saugleitung (3) benachbart zur zugehörigen Brusthaube (1) oder einer Steckverbindung zwischen Saugleitung (3) und Brusthaube (1) angeordnet ist.

8. Brustpumpenset nach einem der Ansprüche 6 oder 7, wobei im Bereich der Brusthaube (1) eine Anzeige zur Anzeige von Informationen vorhanden ist, wobei der Informationsfluss von der Signalempfängereinheit (5) zur Anzeige erfolgt, wobei die Anzeige vorzugsweise eine optische und/oder akustische Anzeige ist.

9. Brustpumpenset nach einem der Ansprüche 6 bis 8, wobei die Signalempfänger- und Sendeeinheit (5) vorzugsweise Bedienungsmittel (51, 52, 53) zur Bedienung der Saugpumpe aufweist, und/oder wobei als Signal von der Signalempfänger- und Sendeeinheit (5) zur Brustpumpe vorzugsweise ein manueller Pumpmodus oder ein mittels des Bedienungsmittels programmierter Pumpmodus übertragbar ist.

10. Brustpumpenset nach einem der Ansprüche 6 bis 9, wobei zwischen Signalempfängereinheit (5) und Saugpumpe eine analoge oder digitale elektrische Leitung (6) vorhanden ist zur Übermittlung der Signale oder wobei die Signalempfängereinheit (5) einen Empfänger und gegebenenfalls einen Sender von bzw. für drahtlos übermittelte(n) Signale(n) aufweist.

11. Brustpumpenset nach einem der Ansprüche 6 bis 10, wobei die Brustpumpe mit einer Signalsende- und gegebenenfalls mit einer Signalempfangseinheit zur Sendung und zum Empfang von Signalen an die brusthaubenseitige Signalempfänger- und Sendeeinheit (5) bzw. von der brusthaubenseitigen Signalempfänger- und Sendeeinheit (5) ausgerüstet ist.

12. Brustpumpenset nach einem der Ansprüche 6 bis 11, wobei zusätzlich zur Saugpumpeneinheit (4) eine externe Einheit (4') vorhanden ist, welche mit einer Signalsendeeinheit zur Sendung von Signalen an die brusthaubenseitige Signalempfängereinheit (5) ausgerüstet ist.

13. Verfahren zur Betreibung eines Brustpumpensystems gemäss einem der Ansprüche 6 bis 12, wobei Signale an eine brusthaubenseitige Signalempfängereinheit (5) übermittelt werden, wobei vorzugsweise eine oder mehrere der nachfolgenden Informationen übermittelt werden: Saugfrequenz, Saugleistung, verwendetes Pumpprogramm, zur Auswahl stehende Pumpprogramme, Pumpdauer, Uhrzeit, Füllstand im Milchsammelbehälter, Brusthaubentemperatur, Milchflussmessung.

14. Verfahren nach Anspruch 13, wobei die Signale von einer Saugpumpeneinheit übermittelt werden.

15. Brusthaube (1) zur Verwendung in einem Brustpumpenset nach einem der Ansprüche 1 bis 3, wobei sie eine Signalsendeeinheit (5) zur Übermittlung von Signalen an eine Saugpumpeneinheit (4) aufweist, wobei die Signalsendeeinheit (5) eine Programmierungsvorrichtung zur manuellen oder automatischen Programmierung der Saugpumpeneinheit (4) umfasst, und **dadurch gekennzeichnet, dass** die Programmierungsvorrichtung dazu ausgebildet ist, einen gespeicherten Pumpvorgang zu übermitteln.

## Claims

1. Breastpump set for expressing human breastmilk, said breastpump set comprising at least one breast shield (1) to be placed on a mother's breast with a coupling part (12) for connection to a milk collection container (2), and a suction pump unit (4) with an electrically operated suction pump, wherein on the side of the breast shield a first signal transmission unit (5) is provided for transmitting signals to the suction pump unit (4), wherein said first signal transmission unit (5) comprises a programming device for manual or automatic programming of the suction pump unit (4), **characterized in that** programming device is designed for transmitting a stored pumping procedure to the suction pump unit (4).

2. Breastpump set according to claim 1, wherein the programming device detects a manual pumping procedure and transmits this to the suction pump unit (4) and/or wherein the programming device activates a stored pumping procedure in the suction pump unit (4).

3. Breastpump set according to one of claims 1 or 2, wherein the first signal transmission unit (5) is designed for wireless transmission of the signals.

4. Breastpump set according to one of claims 1 to 3, wherein the breastpump set comprises two signal transmission units, of which the first signal transmission unit (5) is provided on the side of the breast shield and a second signal transmission unit is provided on the side of the suction pump.

5. Breastpump set according to claim 4, wherein the two signal transmission units are designed for wireless transmission of signals and/or wherein the signal transmission unit (5) on the side of the breast shield is arranged on the breast shield (1), on a plug connection on the side of the breast shield of a suction tube (3) between breast shield (1) and suction pump (4), or on the suction tube (3).

6. Breastpump set according to one of claims 1 to 5, wheein a signal reception unit (5) is arranged in the area of the breast shield (1).

7. Breastpump set according to claim 6, wherein the signal reception unit (5) is arranged on or in the breast shield (1) or wherein for each breast shield (1) a suction line (3) is present for connecting the suction pump (4) to the at least one breastpump, and wherein the signal reception unit (5) is arranged on the suction line (3) adjacent to the associated breast shield (1) or on a plug connection between suction line (3) and breast shield (1).

8. Breastpump set according to one of claims 6 or 7, wherein a display for displaying information is present in the area of the breast shield (1), the flow of information being from the signal reception unit (5) to the display, weherein the the display is preferably an optical and/or acoustic display.

9. Breastpump set according to one of claims 6 through 8, wherein the signal reception and signal transmission unit (5) prerferably has operating means (51, 52, 53) for operating the suction pump and/or wherein preferably a manual pump mode, or a pump mode programmed by the operating means, can be transmitted as signal from the signal reception and transmission unit (5) to the breastpump.

10. Breastpump set according to one of claims 6 through 9, wherein an analog or digital electrical lead (6) is present between signal reception unit (5) and suction pump for transmitting the signals or wherein the signal reception unit (5) has a receiver for wirelessly transmitted signals and if applicable a transmitter for transmitting wirelessly transmitted signal(s).

11. Breastpump set according to one of claims 6 through 10, wherein the breastpump is equipped with a signal transmission unit and if applicable with a signal receiver unit for transmitting and receiving signals to or from the signal reception and transmission unit (5) on the side of the breast shield respectively.

12. Breastpump set according to one of claims 6 through 11, wherein, in addition to the suction pump unit (4), an external unit (4') is present that is equipped with a signal transmission unit for transmitting signals to the signal reception unit (5) on the side of the breast shield.

13. Method for operating a breastpump system according to one of claims 6 through 12, wherein signals are transmitted to a signal reception unit (5) on the side of the breast shield, wherein preferably one or more of the following information items are transmitted: suction frequency, suction capacity, pump program used, pump programs available for selection, pump duration, clock time, filling level in the milk collection container, breast shield temperature, milk flow measurement.

14. Method according to claim 13, wherein the signals are transmitted from a suction pump unit.

15. Breast shield (1) for use in a breastpump set according to one of claims 1 to 3, wherein the breast shield comprises a signal transmission unit (5) for transmitting signals to a suction pump unit (4), wherein the signal transmission unit (5) comprises a programming device for manual or automatic programming of the suction pump unit (4), **characterized in that** the programming device is designed for transmitting a stored pumping procedure.

## Revendications

1. Ensemble tire-lait pour soutirer du lait maternel humain, l'ensemble tire-lait présentant au moins une téterelle (1) destinée à être appliquée sur un sein maternel et munie d'une partie d'accouplement (12) destinée à être reliée avec un récipient de collecte de lait (2) et une unité à pompe de succion (4) munie d'une pompe de succion à entraînement électrique, une première unité d'émission de signaux (5) étant disposée du côté de la téterelle pour la transmission de signaux à l'unité à pompe de succion (4) et cette première unité d'émission de signaux (5) comprenant un dispositif de programmation pour la programmation manuelle ou automatique de l'unité à pompe de succion (4), **caractérisé en ce que** le dispositif de programmation est configuré pour transmettre une opération de pompage mise en mémoire à l'unité à pompe de succion (4).

2. Ensemble tire-lait selon la revendication 1, avec lequel le dispositif de programmation détecte une opération de pompage manuelle et transmet celle-ci à l'unité à pompe de succion (4) et/ou avec lequel le dispositif de programmation active une opération de pompage mise en mémoire dans l'unité à pompe de succion (4).

3. Ensemble tire-lait selon la revendication 1 ou 2, avec lequel la première unité d'émission de signaux (5) est configurée pour la transmission sans fil des signaux.

4. Ensemble tire-lait selon l'une des revendications 1 à 3, avec lequel l'ensemble tire-lait présente deux unités d'émission de signaux et avec lequel la première unité d'émission de signaux (5) est disposée du côté de la téterelle et une deuxième unité d'émission de signaux du côté de la pompe de succion.

5. Ensemble tire-lait selon la revendication 4, avec lequel les deux unités d'émission de signaux sont configurées pour la transmission sans fil des signaux et/ou avec lequel l'unité d'émission de signaux (5) du côté de la téterelle est disposée de préférence sur la téterelle (1), sur un connecteur côté téterelle vers un tuyau de succion (3) entre la téterelle (1) et la pompe de succion (4) ou sur le tuyau de succion (3).

6. Ensemble tire-lait selon l'une des revendications 1 à 5, avec lequel une unité de réception de signaux (5) est disposée dans la zone de la téterelle (1).

7. Ensemble tire-lait selon la revendication 6, avec lequel l'unité de réception de signaux (5) est disposée sur ou dans la téterelle (1) et il existe pour chaque téterelle (1) une conduite de succion (3) pour relier la pompe de succion (4) avec l'au moins un tire-lait et avec lequel l'unité de réception de signaux (5) est disposée sur la conduite de succion (3) à côté de la téterelle (1) associée ou d'un connecteur entre la conduite de succion (3) et la téterelle (1).

8. Ensemble tire-lait selon l'une des revendications 6 ou 7, avec lequel il existe dans la zone de la téterelle (1) un indicateur pour indiquer des informations, la circulation des informations s'effectuant de l'unité de réception de signaux (5) vers l'indicateur, l'indicateur étant de préférence un indicateur visuel et/ou sonore.

9. Ensemble tire-lait selon l'une des revendications 6 à 8, avec lequel l'unité d'émission et de réception de signaux (5) présente de préférence des moyens de commande (51, 52, 53) pour commander la pompe de succion et/ou avec lequel le signal qui peut être transmis de l'unité d'émission et de réception de signaux (5) vers le tire-lait est de préférence un mode de pompage manuel ou un mode de pompage programmé par le biais du moyen de commande.

10. Ensemble tire-lait selon l'une des revendications 6 à 9, avec lequel il existe entre l'unité de réception de signaux (5) et la pompe de succion une ligne électrique (6) analogique ou numérique pour la transmission des signaux ou avec lequel l'unité de réception de signaux (5) présente un récepteur et éventuellement un émetteur de ou pour un ou plusieurs signaux transmis sans fil.

11. Ensemble tire-lait selon l'une des revendications 6 à 10, avec lequel le tire-lait est équipé d'une unité d'émission de signaux et éventuellement d'une unité de réception de signaux pour émettre et recevoir des signaux à l'unité d'émission et de réception de signaux (5) côté téterelle ou de la part de l'unité d'émission et de réception de signaux (5) côté téterelle.

12. Ensemble tire-lait selon l'une des revendications 6 à 11, avec lequel il existe en plus de l'unité à pompe de succion (4) une unité externe (4'), laquelle est équipée d'une unité d'émission de signaux pour émettre des signaux à l'unité de réception de signaux (5) côté téterelle.

13. Procédé pour faire fonctionner un système tire-lait selon l'une des revendications 6 à 12, selon lequel des signaux sont transmis à une unité de réception de signaux (5) côté téterelle, une ou plusieurs des informations suivantes étant de préférence transmises : fréquence de succion, puissance de succion, programme de pompage utilisé, programmes de pompage disponibles à la sélection, durée du pompage, heure, niveau de remplissage du récipient de collecte de lait, température de la téterelle, mesure du débit de lait.

14. Procédé selon la revendication 13, selon lequel les signaux sont transmis depuis une unité à pompe de succion.

15. Téterelle (1) destinée à être utilisée dans un ensemble tire-lait selon l'une des revendications 1 à 3, celle-ci présentant une unité d'émission de signaux (5) pour la transmission de signaux à une unité à pompe de succion (4), l'unité d'émission de signaux (5) comprenant un dispositif, de programmation pour la programmation manuelle ou automatique de l'unité à pompe de succion (4), et **caractérisée en ce que** le dispositif de programmation est configuré pour transmettre une opération de pompage mise en mémoire.
